# EUROPÄISCHE PATENTSCHRIFT

(11) **EP 1 669 035 B1**
(45) Veröffentlichungstag und Bekanntmachung des Hinweises auf die Patenterteilung: **05.03.2014**
(21) Anmeldenummer: 05024636.2
(22) Anmeldetag: 11.11.2005
(51) Int. Cl.: A61B 17/16

(54) **Vorrichtung zum Räumen von Knochenhöhlen**
Device for cleaning out bone cavities
Dispositif pour curer des cavités osseuses

(30) Priorität: 10.12.2004 DE 202004019105 U
(43) Veröffentlichungstag der Anmeldung: 14.06.2006
(73) Patentinhaber: Stryker Trauma GmbH, 24232 Schönkirchen (DE)
(72) Erfinder: Windhager, Reinhard, Dr. Prof. Univ., 8036 Graz (AT); Prager, Ronald, 24796 Bovenau (DE); Robioneck, Bernd, 24111 Preetz (DE)
(74) Vertreter: Maiwald Patentanwalts GmbH

(56) Entgegenhaltungen:
- US-A- 4 043 037
- US-A- 5 628 747
- US-A1- 2002 183 758
- US-A1- 2003 083 681

## Beschreibung

Die Erfindung bezieht sich auf eine Vorrichtung zum Räumen von Knochenhöhlen nach dem Anspruch 1.

Insbesondere bei tumorbefallenen Knochen ergibt sich die Notwendigkeit, das befallende Material zu entfernen. Hierzu ist bekannt, den befallenden Knochenabschnitt auszulöffeln. Ein derartiges Verfahren ist aufwendig und erfordert den Zutritt zum Behandlungsort über Fleisch und Gewebe.

Es ist ferner bekannt, mit Hilfe eines Bohrers bzw. eines Räumwerkzeugs einen Röhrenknochen axial zu räumen und das aufgelockerte Material abzusaugen. Das Aufbohren führt jedoch zu einer starken Belastung der Kortikalis aufgrund des auftretenden Druckes.

Aus US-A-2003/008368 ist eine Vorrichtung bekannt mit den Merkmalen des Oberbegriffs des Anspruchs 1.

Aus US-A-2002/0183758 ist ein flexibles Element bekannt, das an seinem einen Ende mit der Stirnseite einer Drehwelle verbunden ist, und an seinem anderen Ende ein Profilelement mit scharfen Kanten trägt zum Entfernen von Knochenmaterial.

Der Erfindung liegt die Aufgabe zugrunde, eine Vorrichtung zum Räumen von Knochenhöhlen, z.B. eines Kanals eines Röhrenknochens, zu schaffen, mit dem der Knochen wirksam innerhalb kürzester Zeit geräumt werden kann, ohne den Knochen zu stark zu belasten.

Diese Aufgabe wird durch die Merkmale des Anspruchs 1 gelöst.

Die erfindungsgemäße Vorrichtung sieht einen länglichen hohlen Schaft vor. Der Schaft kann über die Länge einen gleichmäßigen Durchmesser haben und an einem Ende eine Erweiterung zwecks drehfester Einspannung in eine Drehantriebsvorrichtung. Der längliche Schaft weist eine axiale Durchbohrung auf, wobei die Durchbohrung über ihre Länge einen konstanten Durchmesser aufweisen kann. Der Schaft kann aus einem geeigneten Metall oder auch aus Kunststoff gefertigt werden.

Der Schaft dient als Lager und Gehäuse für eine Welle, die drehbar im Schaft gelagert ist. Vorzugsweise ist dafür gesorgt, daß die Welle im Schaft axial festgelegt ist. Das eine Ende der Welle erstreckt sich zum Einspannende des Schaftes hin und ist so ausgebildet, das es in Drehverbindung mit einer Antriebsspindel einer Drehantriebsvorrichtung gebracht werden kann. Das andere Ende der Welle erstreckt sich über das zugehörige Ende des Schaftes hinaus. Mit diesem freien Ende der Welle ist seitlich ein längliches flexibles Element verbunden, insbesondere aus Kunststoff oder Metall z.B. in Form eines Fadens oder eines Seils. Mit dem anderen Ende des flexiblen Elements ist ein Profilelement aus Metall verbunden.

Im Betrieb wird eine derartige Vorrichtung oder ein derartiges Werkzeug mit dem einen Ende des Schaftes in eine Drehantriebsvorrichtung eingespannt. Diese kann so ausgebildet sein, daß ihre Antriebsspindel automatisch in Drehverbindung mit dem zugeordneten Ende der Welle gelangt. Die Drehantriebsvorrichtung dient zugleich zur Führung des erfindungsgemäßen Werkzeugs. Wird die Drehantriebsvorrichtung in Betrieb gesetzt, dreht sich die Welle und das Profilelement wird auf diese Weise im Kreis um die Achse der Welle geschleudert. Das Profilelement, das vorzugsweise aus Metall geformt und mit scharfen Kanten ausgebildet ist, räumt die Spongiosa des Knochens und lockert das spongiöse Material auf, so daß es mit Hilfe geeigneter Vorkehrungen aus dem Knochen entfernt werden kann, z.B. mit geeigneten Saugmitteln. Es versteht sich, daß eine Saugvorrichtung auch in das erfindungsgemäße Werkzeug integriert werden kann.

Zur Befestigung des flexiblen Elements sieht die Erfindung vor, daß das zweite Ende der Welle eine Querbohrung aufweist zur Aufnahme des zugeordneten Endes des flexiblen Elements. Eine Befestigungsschraube wird vom freien Ende der Welle in eine Gewindebohrung eingeschraubt und klemmt das zugeordnete Ende des flexiblen Elements in der Querbohrung fest. Es versteht sich, daß unterschiedlich lang eingespannte flexible Elemente oder unterschiedlich lange flexible Elemente verwendet werden können zur Anpassung an den zu räumenden Durchmesser im Knochen.

Das flexible Element kann aus Kunststoff oder aus Metall bestehen. Die Welle kann flexibel sein, und es kann ein Satz unterschiedlicher langer Wellen vorgesehen werden zur Anpassung an unterschiedliche zu räumende Längen.

Die Erfindung wird nachfolgend anhand eines Ausführungsbeispiels näher erläutert.
- Fig. 1: zeigt in Seitenansicht eine Vorrichtung nach der Erfindung.
- Fig. 2: zeigt die Vorrichtung nach Fig. 1 in einer um 90° gedrehten Position.

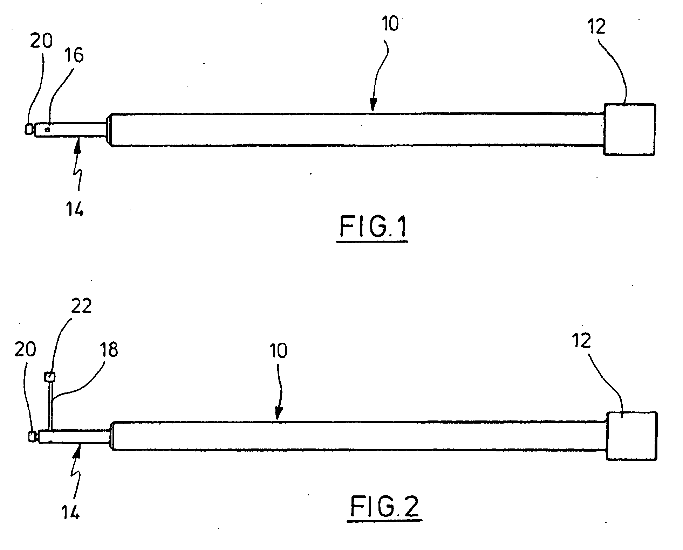

In den Fign. 1 und 2 ist ein länglicher Schaft 10 zu erkennen, der im Querschnitt kreisförmig ist und über den größten Teil seiner Länge einen konstanten Außendurchmesser aufweist. Am rechten Ende weist der Schaft 10 einen erweiterten Abschnitt 12 auf. Dieser kann, was nicht gezeigt ist, drehfest in eine nicht gezeigte Drehantriebsvorrichtung eingespannt werden. Der Schaft 10 weist durchgehend eine axiale Bohrung auf (nicht zu sehen). Diese Durchbohrung nimmt eine Welle 14 auf, die sich in den Fign. 1 und 2 über den größten Teil ihrer Länge in dem Schaft 10 erstreckt, und zwar bis zum Abschnitt 12. Das rechte Ende der Welle 14 ist so geformt (nicht gezeigt), daß es in Drehverbindung mit einer nicht gezeigten Antriebsspindel einer Drehantriebsvorrichtung koppelbar ist. Das linke Ende der Welle 14 erstreckt sich über das linke Ende des Schaftes 10 hinaus. Im Endbereich weist der freie Abschnitt der Welle 14 eine Querbohrung 16 auf. Sie nimmt ein zugeordnetes Ende eines flexiblen Elements 18 auf. Vom freien Ende des freien Endabschnitts der Welle 14 ist axial eine Gewindebohrung eingeformt (nicht zu sehen), in die eine Feststellschraube 20 eingeschraubt ist. Mit Hilfe der Feststellschraube 20 wird das flexible Elemente in der Querbohrung 16 festgeklemmt.

Wird die in den Fign. 1 und 2 dargestellte Vorrichtung in eine Drehantriebsvorrichtung eingespannt und wird diese in Betrieb gesetzt, dreht die Welle 14 in dem Schaft 10 und schleudert dadurch ein Profilelement 22 am anderen Ende des flexiblen Elements 18 in eine Ebene senkrecht zur Zeichenebene. Das Profilelement 22, das aus Metall besteht und mit relativ scharfen Kanten versehen ist, räumt dadurch eine Knochenhöhle oder einen Knochenkanal, wenn die gezeigte Vorrichtung in z.B. die tumorbedingte Knochenhöhle oder den Knochenkanal eingeführt wird.

## Patentansprüche

1. Vorrichtung zum Räumen einer Knochenhöhle oder eines Kanals eines Röhrenknochens mit:
einem länglichen hohlen Schaft (10), dessen eines Ende zur drehfesten Einspannung in eine Drehantriebsvorrichtung ausgebildet ist,
einer Welle (14), die drehbar im Schaft (10) gelagert ist und mit einem ersten Ende zum Einspannende des Schaftes (10) hin und mit dem zweiten Ende über das andere Ende des Schaftes (10) hinaus erstreckt ist, wobei das erste Ende der Welle (14) zur Drehverbindung mit einer Antriebsspindel der Drehantriebsvorrichtung ausgebildet ist, und
einem flexiblen länglichen Element (18), das an seinem einen Ende seitlich mit dem zweiten Ende der Welle (14) verbindbar ist, **gekennzeichnet durch**
einem Profilelement (22) am anderen Ende des flexiblen Elements (18), wobei das Profilelement (22) aus Metall geformt ist und scharfen Kanten aufweist, und
wobei das zweite Ende der Welle (14) eine Querbohrung (16) aufweist zur Aufnahme des Endes des flexiblen Elements (18) und eine Befestigungsschraube (20) vom freien Ende der Welle (14) axial in eine Gewindebohrung einschraubbar ist zum Festklemmen des Endes des flexiblen Elements (18) in der Querbohrung (16).

2. Vorrichtung nach Anspruch 1, **dadurch gekennzeichnet, dass** das flexible Element (18) ein Faden oder ein Seil ist.

3. Vorrichtung nach einem der Ansprüche 1 bis 3, **dadurch gekennzeichnet, dass** das flexible Element aus Kunststoff oder Metall besteht.

4. Vorrichtung nach einem der Ansprüche 1 bis 3, **dadurch gekennzeichnet, dass** ein Satz verschieden langer Wellen (14) vorgesehen ist.

5. Vorrichtung nach einem der Ansprüche 1 bis 4, **dadurch gekennzeichnet, dass** die Welle (14) zumindest über einen Teil ihrer Länge flexibel ist.

## Claims

1. Device for cleaning out bone cavities or a channel in a long bone, having:
an elongated hollow shaft (10), one end of which is formed such that it is able to be clamped non-rotatably in a rotary drive device,
a mandril (14) that is mounted rotatably in the shaft (10) and the first end of which extends toward the clamped end of the shaft (10), and the second end of which protrudes beyond the other end of the shaft (10), wherein the first end of the mandril (14) is formed so as to allow rotatable connection to a driving spindle of the rotary drive device, and
a flexible elongated element (18), one end of which is connectable to the side of the second end of the mandril (14), **characterised by**
a profile element (22) on the other end of the flexible element (18), wherein the profile element (22) is shaped from metal and has sharp edges, and
wherein the second end of the mandril (14) comprises a cross bore (16) for accommodating the end of the flexible element (18) and wherein a fastening screw (20) is screwable from the exposed end of the mandril (14) axially into a threaded hole to clamp the end of the flexible element (18) firmly inside the cross bore (16).

2. Device according to claim 1, **characterised in that** the flexible element (18) is a thread or a wire.

3. Device according to any of claims 1 to 3, **characterised in that** the flexible element is made from plastic or metal.

4. Device according to any of claims 1 to 3, **characterised in that** a set of mandrils (14) of different lengths is provided.

5. Device according to one of claims 1 to 4, **characterised in that** the mandril (14) is flexible at least over a section of its length.

## Revendications

1. Dispositif destiné à dégager une cavité osseuse ou un canal d'un os long, comprenant :
une tige creuse oblongue (10), dont une extrémité est configurée pour le serrage résistant à la torsion dans un dispositif d'entraînement en rotation,
un axe (14), monté tournant dans la tige (10) et qui s'étend par une première extrémité en direction de l'extrémité de serrage de la tige (10) et par la seconde extrémité au-delà de l'autre extrémité de la tige (10), la première extrémité de l'axe (14) étant configurée pour l'assemblage tournant avec une broche d'entraînement du dispositif d'entraînement en rotation, et
un élément oblong flexible (18), qui peut être raccordé latéralement à l'une de ses extrémités à la seconde extrémité de l'axe (14), **caractérisé par**
un élément profilé (22) à l'autre extrémité de l'élément flexible (18), l'élément profilé (22) étant formé de métal et présentant des arêtes vives, et
la seconde extrémité de l'axe (14) présentant un alésage transversal (16) pour recevoir l'extrémité de l'élément flexible (18) et une vis de fixation (20) pouvant être vissée de l'extrémité libre de l'axe (14), dans la direction axiale, dans un taraudage pour le serrage de l'extrémité de l'élément flexible (18) dans l'alésage transversal (16).

2. Dispositif selon la revendication 1, **caractérisé en ce que** l'élément flexible (18) est un fil ou un câble.

3. Dispositif selon l'une des revendications 1 à 3, **caractérisé en ce que** l'élément flexible se compose de matière plastique ou de métal.

4. Dispositif selon l'une des revendications 1 à 3, **caractérisé en ce qu'**il est prévu un jeu d'axes (14) de longueurs différentes.

5. Dispositif selon l'une des revendications 1 à 4, **caractérisé en ce que** l'axe (14) est flexible au moins sur une partie de sa longueur.
